(19) Europäisches Patentamt
European Patent Office
Office européen des brevets

(11) **EP 3 622 984 A1**

(12) **EUROPÄISCHE PATENTANMELDUNG**

(43) Veröffentlichungstag:
**18.03.2020 Patentblatt 2020/12**

(51) Int Cl.:
*A61M 5/168* *(2006.01)*    *G01F 1/36* *(2006.01)*

(21) Anmeldenummer: **19020494.1**

(22) Anmeldetag: **28.08.2019**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(30) Priorität: **11.09.2018 CH 10732018**

(71) Anmelder: **ReseaTech GmbH
3400 Burgdorf (CH)**

(72) Erfinder:
• **Zumbrunnen, Simon
  3414 Oberburg (DE)**
• **Haslebacher, Philipp
  3400 Burgdorf (DE)**

(54) **CHEMISCH RESISTENTE DURCHFLUSSMESSVORRICHTUNG FÜR FLÜSSIGKEITEN**

(57)    - Die Erfindung betrifft eine Durchflussmessvorrichtung für Flüssigkeiten auf dem Prinzip einer Differenzdruckmessung mit austauschbaren Strömungswiderständen sowie durch ein Elastomer geschützten Druckmesszellen.

## Fig. 1

EP 3 622 984 A1

## Beschreibung

### Umfeld:

**[0001]**    - In vielen Industriezweigen werden kleine Flüssigkeitsmengen mittels Magnetventilen oder Pumpen dosiert. Zur Überwachung dieser Systeme wird dabei auf optische, kapazitive oder auch gravimetrische Systeme gesetzt. Eine weitere Möglichkeit ist die Messung kleiner Volumina durch einen Inline-Durchflusssensor. Diese Inline-Messung funktioniert jedoch häufig aufgrund des relativ trägen Ansprechverhaltens herkömmlicher Durchflussmessgeräte erst bei Dosiermengen von mehreren Millilitern und Dosierzeiten von mehreren Sekunden. In der Mikrodosiertechnik werden aber häufig Tropfen im Grössenbereich von Nano- bis Mikroliter dosiert. Solche Tropfengrössen lassen nur ein Zeitfenster von wenigen Millisekunden für die Durchflussmessung offen, was hohe Anforderungen an das Messsystem stellt.

**[0002]**    - Diese Erfindung beschreibt deshalb einen Sensor der durch seine kurze Ansprechzeit und kompakte Bauweise kleinste Flüssigkeitsmengen im Nano-bis Milliliter Bereich messen kann. Weiter eignet sich ein solcher Sensor aufgrund seiner kurzen Ansprechzeit für die Überwachung stark pulsierender Pumpen, wie zum Beispiel Piezomembran- oder Peristaltik Pumpen.

### Stand der Technik:

### Durchflussmessung

**[0003]**    - Die Durchflussmessung mittels zwei oder mehr Druckmesskammern die jeweils durch einen Strömungswiderstand fliessend miteinander verbunden sind wurde bereits 1989 in EP 0337092 der Firma Labionics AG beschrieben und ist heute Stand der Technik. EP 0337092 schlägt insbesondere eine Trennung der Druckmesszellen vom Fluid vor, um diese vor chemisch aggressiven, giftigen oder radioaktiven Stoffen zu schützen. Die Entkopplung der Druckmesszellen vom Fluid führt aber in jedem Fall auch zu einem trägeren Ansprechverhalten, was das Messen von dynamischen Prozessen verunmöglicht.

**[0004]**    - Kleine Durchflusssensoren die auf dem Prinzip der Differenzdruckmessung basieren kombinieren oft Kanal, Restriktor und Druckmesszellen in einer Einheit (siehe DE 19650115 und US6898981). Dieser Aufbau ist sehr kompakt, macht jedoch die Änderung der Restriktordimensionen und dadurch die Anpassung an verschiedene Durchflussbereiche aufwändig. Zudem sind die Druckmesszellen in direkter Berührung mit dem Medium, was das Messen von korrosiven Flüssigkeiten und Gasen verunmöglicht, weil diese Sensoren durch ihren Aufbau im MEMS-Verfahren in der Werkstoffwahl stark eingeschränkt sind.

**[0005]**    - Grosse Durchflusssensoren die auf dem Prinzip der Differenzdruckmessung basieren (Bsp: US6463810), sind entweder mit zwei Druckmesszellen ausgestattet, welche ortsunabhängig montiert und kontaktiert sind, oder sie verwenden eine einzige Differenzdruckmesszelle, die über Kanäle mit dem Medium verbunden ist.

**[0006]**    - Die hier beschriebene Erfindung erreicht einen kompakten Aufbau durch die Anordnung zweier Druckmesszellen auf einer Leiterplatte und mit direkter Ankoppelung an den Kanal. Die Werkstoffwahl ist dabei nicht eingeschränkt.

### Druckmessung

**[0007]**    - Bei Drucksensoren für medizinische, und biotechnologische Einwegapplikationen (NPX 2300, Nova NPC-100) ist häufig ein elastisches, jedoch inkompressibles Medium auf der Druckmesszelle aufgebracht. Dieses Kopplungsmedium überträgt praktisch verzögerungsfrei den Flüssigkeitsdruck auf die Druckmesszelle und schützt gleichzeitig die Messzelle vor Lösungsmitteln oder chemisch ätzenden Medien. Als Kopplungsmedium werden für diese Einwegsensoren oft fluorierte Silikone mit sehr niedrigen Härten (< 20 Shore 00) verwendet.

### Erklärung der Erfindung:

**[0008]**    - Die vorliegende Erfindung verbindet das System der durch Kopplungsmedien geschützten, medizinischen Druckmesszellen mit dem Differenzdruck-Durchflussmessverfahren. Dadurch entsteht ein chemisch resistenter Volumenstromsensor.

**[0009]**    Durch die sehr kurze und direkte Ankopplung der Drucksensoren an die Flüssigkeit wird ein hoch dynamisches Ansprechverhalten erzielt. Weiter kann durch den direkten Einbau der Druckmesszellen eine sehr kompakte und kleine Bauweise erreicht werden, ohne Einschränkung in der Werkstoffwahl.

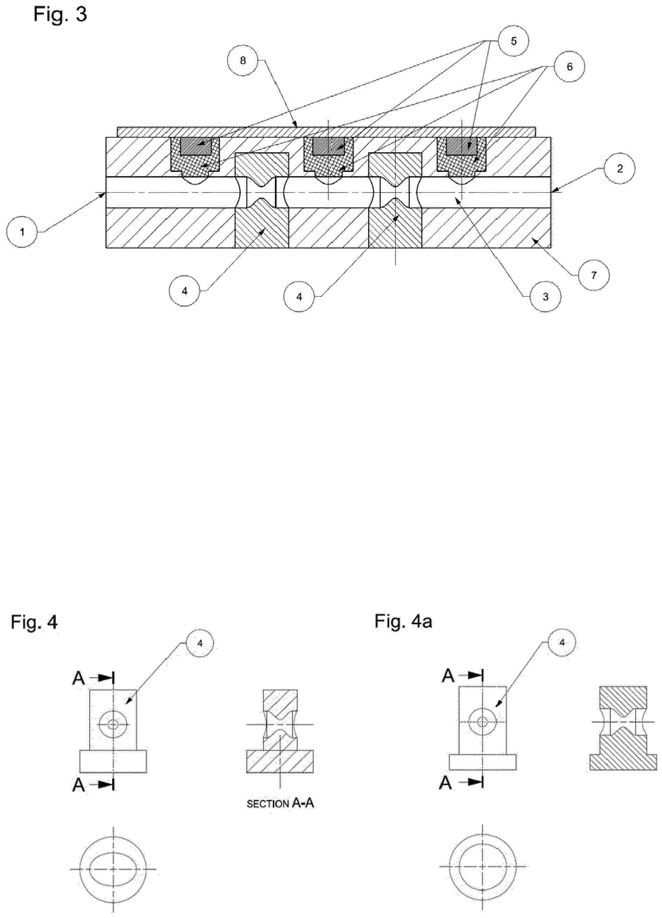

**[0010]**    - Die Erfindung ist dadurch gekennzeichnet, dass sich ein oder mehrere Restriktoren (Fig. 3) durch form- und/oder kraftschlüssige Verbindungen Ein- und Ausbauen lassen. Dies dient zum Einstellen des gewünschten Messbereiches sowie zur Wartung des Sensors.

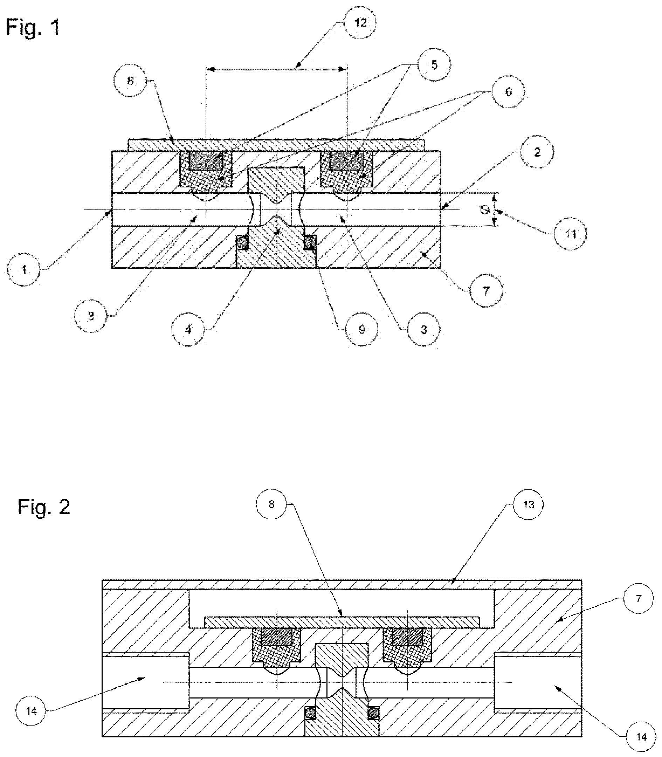

**[0011]**    - Weiter schlägt die vorliegende Erfindung die Montage aller Druckmesszellen (5) und deren elektrische Kontaktierung auf derselben Leiterplatte (8) vor. Die Druckmesszellen befinden sich dabei auf der dem Messkanal zugewandten Leiterplattenseite wie es in Fig.1 ersichtlich ist.

### Worterklärungen

**[0012]**    - Messkammer (3): Die Beschreibung der Erfindung benutzt das Wort Messkammer. Damit ist ein Volumen mit rundem, eckigen oder auch flachem Querschnitt gemeint, in welchem der Stömungsquerschnitt deutlich grösser und somit die Strömungsgeschwindigkeit deutlich niedriger ist als im Restriktor. Die Messkammer kann dabei auch ein Abschnitt in der Rohrleitung ohne Querschnittsveränderung sein.

**[0013]** - Restriktor (4): Das Wort «Restriktor» beschreibt eine lokale, deutliche Verkleinerung des Strömungsquerschnitts über eine bestimmte Strecke. Der Restriktor kann in Form einer Kapillare, Lochblende, eines Netzes oder eines anderen Objekts im Kanal ausgebildet sein und führt sowohl zu einer Umlenkung wie auch zu Reibungseffekten in der strömenden der Flüssigkeit. Der dadurch entstehende Druckabfall ($\Delta p_v$) wird in der Literatur beispielsweise bei einem kapillarförmigen Restriktor wie folgt beschrieben:

$$\Delta p_v = \frac{128 * \eta * l}{\pi * d^4} * \dot{V} + \frac{\zeta_2 * \rho * 8}{d^4 * \pi^2} * \dot{V}^2$$

**[0014]** Dabei ist $\eta$ die Viskosität der Flüssigkeit, $\zeta_2$ eine Geometriekonstante die die Querschnittsveränderung beschreibt, $\dot{V}$ der Volumenstrom, $\rho$ die Dichte der Flüssigkeit, d der Durchmesser und l die Länge des Restriktors.

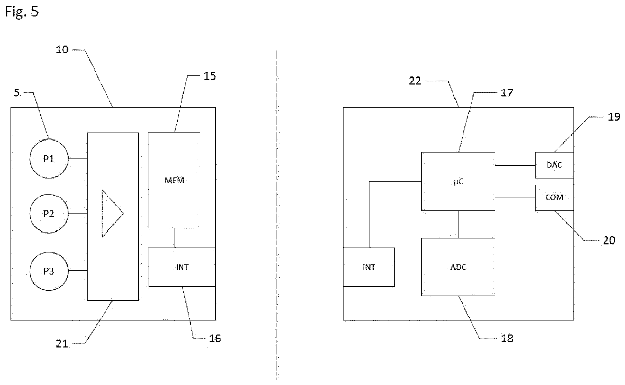

**[0015]** Legende zu den Zeichnungen Fig.1 bis Fig.5:

1. Flüssigkeitszulauf (/-ablauf bei Rückwärtsfluss)
2. Flüssigkeitsablauf (/-zulauf bei Rückfluss)
3. Messkammern (für Druckmessung)
4. Restriktor
5. Druckmesszelle
6. Elastisches Medium (Kopplungsmedium)
7. Kanal / Kanal und Gehäuse
8. Leiterplatte
9. Dichtungsring
10. Durchflussmessvorrichtung
11. Kanaldurchmesser
12. Sensorabstand
13. Deckel
14. Fluidanschlüsse
15. Speichereinheit
16. Schnittstelle
17. Volumenstromberechnung (Mikrocontroller)
18. Analog-/Digital Wandler
19. Digital-/Analog Wandler
20. Digitale Schnittstelle
21. Drucksignalverstärkung
22. Auswerteelektronik

**Patentansprüche**

1. Eine Durchflussmessvorrichtung für Flüssigkeiten bestehend aus einer Messstrecke und einem Messwandler zwischen einem Flüssigkeitszu- (1) und -ablauf (2), **dadurch gekennzeichnet, dass** die Messstrecke mindestens zwei hintereinander angeordnete Messkammern (3) aufweist, die jeweils über einen definierten Restriktor (4) miteinander verbunden sind. Weiter ist jeder Messkammer eine Druckmesszelle (5) zugeordnet, die eines dem Druck in der Messkammer entsprechendes Messsignal erzeugt. Dabei ist jede Druckmesszelle durch ein elastisches Medium (6) von der Flüssigkeit abgekapselt (Fig. 1).

2. Vorrichtung nach Anspruch 1, **dadurch gekennzeichnet, dass** der definierte Restriktor (4) durch eine lösbare, form- oder kraftschlüssige Verbindung eingebaut ist.

3. Vorrichtung nach Anspruch 2, **dadurch gekennzeichnet, dass** der Restriktor kraftschlüssig, mittels Übermasspassung eingebaut ist und über einen runden und oder ovalen Querschnitt nach Fig. 4 oder Fig. 4a verfügt.

4. Vorrichtung nach Anspruch 2, **dadurch gekennzeichnet, dass** der Restriktor als Lochblende mit rundem Querschnitt geformt ist (Fig. 4).

5. Vorrichtung nach Anspruch 2, **dadurch gekennzeichnet, dass** der Kanal (7) oder der Restriktor (4) oder beide aus Kunststoff hergestellt sind.

6. Vorrichtung nach Anspruch 1, **dadurch gekennzeichnet, dass** die Druckmesszellen (5) auf einer einzigen, dem Messkanal gegenüberliegenden Leiterplatte (8) befestigt und auf derselben elektrisch kontaktiert sind.

7. Eine Vorrichtung gemäss Anspruch 6, **dadurch gekennzeichnet, dass** die Drucksignalverstärkung (21) auf der Durchflussmessvorrichtung (10) (Fig.5), die Analog/Digital-Wandlung (18) und Volumenstromberechnung (17) jedoch räumlich trennbar von der Messstrecke aufgebaut sind.

8. Eine Vorrichtung gemäss Anspruch 6, **dadurch gekennzeichnet, dass** die Kalibrierdaten der Durchflussmessvorrichtung (10) in einer elektrischen Speichereinheit (15) auf der Leiterplatte (8) gespeichert sind.

9. Vorrichtung nach Anspruch 2, **dadurch gekennzeichnet, dass** der Sensorabstand (12) weniger als 50 mm und der Kanaldurchmesser (11) weniger als 10 mm beträgt.

10. Vorrichtung nach Anspruch 1, **dadurch gekennzeichnet, dass** der Kanal (7) sowohl das Gehäuse der Durchflussmessvorrichtung als auch deren Fluidanschlüsse (14) darstellt (Fig. 2).

Fig. 1

Fig. 2

Fig. 3

Fig. 4

SECTION A-A

Fig. 4a

Fig. 5

Europäisches Patentamt

European Patent Office

Office européen des brevets

# EUROPÄISCHER RECHERCHENBERICHT

Nummer der Anmeldung

EP 19 02 0494

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2009/093774 A1 (WANG JONG H [US] ET AL) 9. April 2009 (2009-04-09) <br> * Absatz [0062]; Abbildung 7 * <br> * Absatz [0061] * <br> * Absatz [0060] * <br> * Absatz [0059] * <br> ----- | 1-5,9,10 | INV. <br> A61M5/168 <br> G01F1/36 |
| X | WO 2016/057982 A1 (NXSTAGE MEDICAL INC [US]) 14. April 2016 (2016-04-14) <br> * Absatz [0114] * <br> ----- | 1 | |
| A | US 2010/137842 A1 (GIBSON SCOTT R [US]) 3. Juni 2010 (2010-06-03) <br> * das ganze Dokument * <br> ----- | 1-10 | |

| RECHERCHIERTE SACHGEBIETE (IPC) |
|---|
| A61M <br> G01F |

Der vorliegende Recherchenbericht wurde für alle Patentansprüche erstellt

| Recherchenort | Abschlußdatum der Recherche | Prüfer |
|---|---|---|
| München | 4. Dezember 2019 | Hausmann, Alexander |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 19 02 0494

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-12-2019

| Im Recherchenbericht angeführtes Patentdokument | Datum der Veröffentlichung | Mitglied(er) der Patentfamilie | Datum der Veröffentlichung |
|---|---|---|---|
| US 2009093774 A1 | 09-04-2009 | KEINE | |
| WO 2016057982 A1 | 14-04-2016 | EP 3204064 A1 | 16-08-2017 |
| | | EP 3539586 A1 | 18-09-2019 |
| | | US 2017296727 A1 | 19-10-2017 |
| | | US 2019231956 A1 | 01-08-2019 |
| | | US 2019231957 A1 | 01-08-2019 |
| | | US 2019231958 A1 | 01-08-2019 |
| | | US 2019240387 A1 | 08-08-2019 |
| | | WO 2016057982 A1 | 14-04-2016 |
| US 2010137842 A1 | 03-06-2010 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

**In der Beschreibung aufgeführte Patentdokumente**

- EP 0337092 A **[0003]**
- DE 19650115 **[0004]**
- US 6898981 B **[0004]**
- US 6463810 B **[0005]**